# EUROPEAN PATENT SPECIFICATION

(11) **EP 1 647 599 B1**
(45) Date of publication and mention of the grant of the patent: **30.04.2008**
(21) Application number: 04742071.6
(22) Date of filing: 21.07.2004
(51) Int. Cl.: C12Q 1/44, G01N 21/64, A61K 31/35, A61P 11/06, A61P 37/08, A61P 35/00

(54) **QUANTITATIVE METHOD FOR DETECTING YESSOTOXINS IN FISHERY PRODUCTS BASED ON THE ACTIVATION CAUSED BY THE TOXIN IN CELLULAR PHOSPHODIESTERASE**
QUANTITATIVES VERFAHREN ZUM NACHWEIS VON YESSOTOXINEN IN FISCHPRODUKTEN AUF GRUNDLAGE DER DURCH DAS TOXIN VERURSACHTEN AKTIVIERUNG ZELLULÄRER PHOSPHODIESTERASE
METHODE QUANTITATIVE DE DETECTION DE YESSOTOXINES DANS DES PRODUITS HALIEUTIQUES BASEE SUR L'ACTIVATION QUE PRODUIT LA TOXINE DANS LES PHOSPHODIESTERASES CELLULAIRES

(30) Priority: 25.07.2003 ES 200301773
(43) Date of publication of application: 19.04.2006
(60) Divisional application: 07017630.0
(73) Proprietor: UNIVERSIDADE DE SANTIAGO DE COMPOSTELA, 15782 Santiago de Compostela (ES)
(72) Inventor: Botana Lopez, L.M., Univ. Santiago de Compostela, 27002 Lugo (ES); Alfonso Rancano, A., Univ. Santiago de Compostela, 27002 Lugo (ES); Pazos Guldris, M.J., Univ. Santiago de Compostela, 27002 Lugo (ES); Rodrigeuz Vieytes, M., Univ. Sant. de Compostela, 27002 Lugo (ES); Loza Garcia, M.I., Univ. Santiago de Compstela, 15782 Santiago de Compostela (ES); Vieites Baptista De Sousa, Juan Manuel, 36310 Vigo (ES)
(74) Representative: Carvajal y Urquijo, Isabel
(86) International application number: PCT/ES2004/000343
(87) International publication number: WO 2005/012543

(56) References cited:
- US-A1- 2003 138 853
- ALFONSO, A. ET AL.: 'A rapid microplate fluorescence method to detect yessotoxins based on their capacity to activate phosphodiesterases' ANAL. BIOCHEM. vol. 326, no. 1, 01 March 2004, pages 93 - 99, XP004488915
- LEIRA, F. ET AL.: 'Characterization of distinct apoptotic changes induced by okadaic acid and yessotoxin in the BE(2)-M17 neuroblastoma cell line' TOXICOLOGY IN VITRO vol. 16, no. 1, February 2002, pages 23 - 31, XP002995442
- ALFONSO, A. ET AL.: 'Determination of phosphodiesterase activity in rat mast cells using the fluorescent cAMP analogue anthraniloyl cAMP' CELLULAR SIGNALLING vol. 7, no. 5, 1995, pages 513 - 518, XP002995440
- RAMSTAD, H. ET AL.: 'Repeatability and validity of a fluorimetric HPLC method in the quantification of yessotoxin in blue mussels (Mytilus edulis) related to the mouse bioassay' TOXICON vol. 39, no. 9, September 2001, pages 1393 - 1397, XP002995443
- ALFONSO, A. ET AL.: 'Yessotoxin, a novel phycotoxin, activates phosphodiesterase activity. Effect of yessotoxin on cAMP levels in human lymphocytes' BIOCHEM. PHARMACOL. vol. 65, no. 2, 15 January 2003, pages 193 - 208, XP002995441
- FLIK, G. ET AL.: "Evidence for the presence of calmodulin in fish mucus" EUROPEAN JOURNAL OF BIOCHEMISTRY, vol. 138, 1984, pages 651-654,

## Description

The present invention describes the detection and quantification of yessotoxins *in vitro* with respect to their ability for activating phosphodiesterase enzymes, one of the cellular targets of these toxins.

Marine phycotoxins are substances produced by algae which represent a serious public health problem. These toxins accumulate in mollusks and fish in such a manner that when they are ingested by man they produce food poisoning. Phycotoxin classification is carried out in five large groups with respect to the type of intoxication they produce: paralyzing toxins (PSP), diarrhetic toxins (DSP), neurotoxic toxins (NSP), ciguatera toxins (CFP) and amnesic toxins (ASP) (Van Dolah, 2000). There are other groups of phycotoxins which produce different symptoms and which are not included in the former groups, which are: pectenotoxins (PTXs), azaspiracids and yessotoxins (YTXs) (Van Dolah, 2000). Initially, PTXs and YTXs were grouped with the DSPs, since they are lipophilic toxins which usually coexist in toxic episodes. However, in the last decade, they have been considered as a different group since they do not induce diarrhea, their oral toxicity is low and their molecules are different (Draisci, Lucentini et al., 2000). Within these last groups, yessotoxins (hereinafter, YTXs) represent a serious economic problem due to their recent and ubiquitous presence, and due to the absence of a sensitive and specific method for detecting them.

YTXs have been detected in Japan, Europe, New Zealand and Chile, and they are produced by the *Protoceratium reticulatum* and *Lingulodinium polyedrum (Gonyaulax polyedra*) dinoflagellates. These toxins accumulate in marine mollusks and are heat stable, and therefore they are not destroyed during the cooking of marine products. Their absorption, from the digestive tube, is low and therefore they are not toxic when ingested orally, although histopathological modifications have been detected in the liver and pancreas after an oral administration of YTXs in rats (Terao, Ito et al., 1990). However, after an intraperitoneal injection these toxins give rise to important cardiotoxic effects and show a high lethal potency (Draisci, Lucentini et al., 2000). These intraperitoneal effects must be taken into account because, as has been mentioned, YTXs coexist with DSP toxins, and when detecting the latter in bioassays they may produce interferences which lead to detecting false positives. For this reason, when preparing fishery product extract for monitoring the presence of these toxins, it is necessary to perform additional extractions with organic solvents which separate YTXs and DSP toxins. Although these modifications imply the extraction of a large amount of fatty acids, which may also give rise to false positives (Yasumoto, Murata et al., 1984).

The detection methods for phycotoxins in samples coming from marine product extracts are classified in assay methods and analytical methods. Assay methods are those which provide a value for the total toxin content by measuring a single biological or biochemical response which encompasses the activity of all the toxins present in the sample. Analytical methods are those in which separation, identification and individual quantification of the toxins in the sample with respect to an instrumental response is performed. The first include *in vivo* assays in rats or mice, and *in vitro* assays, amongst which must be stressed the enzymatic inhibition assays, cell assays, receptor assays, etc. In these cases, toxicity determination is performed with respect to a dose-response curve obtained with one of the representative toxins of each group. Quantification of the response is performed, amongst others, by means of colorimetric, fluorimetric, luminescence, polarized fluorescence methods, or by determining ligand-receptor interactions in real time with biosensors. The second are *in vitro* assays requiring a prior calibration of the instrumental equipment with standards of known concentrations of each toxin. These assays include chemical methods such as high performance liquid chromatography (HPLC), mass spectrometry or capillary electrophoresis. In general, the instrumental chemical methods are used when it is necessary to identify and quantify each one of the toxins present in a sample. However, in monitoring or health inspection programs a greater relevance is given to knowledge of the potential overall toxicity and therefore assay methods, also called functional methods, are used (Fernández, Míguez et al., 2002).

There are several liquid chromatography-mass spectrometry (Draisci, Palleschi et al., 1999; Goto, lgarashi et al., 2001) and fluorescence HPLC (Ramstad, Larsen et al., 2001; Yasumoto and Takizawa, 1997) analytical methods for detecting YTXs in contaminated mollusks. Within the *in vitro* functional assays for detecting these toxins there are two recent ones: the E-cadherin detection assay (Pierotti, Malaguti et al., 2003; Rossini, 2002) and the caspase activation assay (Malaguti, Ciminello et al., 2002). However, the only officially accepted method is the mouse bioassay, according to Commission Decision 2002/225/EEC of 15 March, 2002. This decision provides that the maximum level of YTXs in a mollusk sample is 1 mg of YTXs per Kg of mollusk flesh. The bioassay consists in observing, for 24 hours, three mice that have been intraperitoneally inoculated with an extract equivalent to 5 grams of mollusk digestive gland, where YTXs usually accumulate, or to 25 grams of whole mollusk. Since the maximum amount of YTX allowed could induce death by intraperitoneal administration within 6 hours, this assay has been modified by shortening the observation time and introducing additional extractions in the method. (Yasumoto, Murata et al., 1984). These extractions allow separating DSP, PTX and azaspiracid toxins from YTXs, although they imply extracting a large amount of fatty acids. If two of the three inoculated mice die, it is considered that there is YTX in the extract. This technique implies sacrificing animals, does not provide an exact value of toxin concentration, is hardly reproducible, gives rise to false positives and needs an additional extraction process in order to be able to discriminate the presence of other toxins. Other biological methods mentioned for detecting YTXs are slow methods with which results are not obtained in less than 24-48 hours and they require a careful process of toxin extraction. Furthermore, they are not based on a specific and unique characteristic of YTXs, since other DSPs are detected along with these toxins.

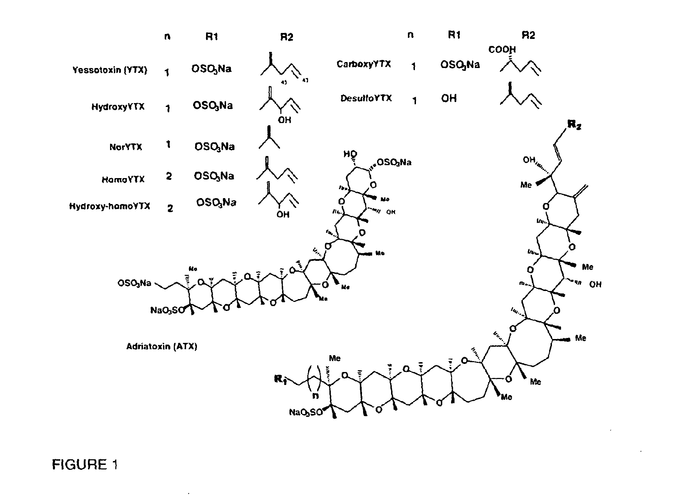

Several studies have been performed in order to determine the cellular target and, therefore, the mechanism of action of YTXs. YTX has a lipophilic molecule consisting of eleven rings with ether groups bound to an unsaturated side chain and to two sulfonic esters. Figure 1 shows some of the natural analogs of YTX which are differentiated in the side chain substituents, although recently more than 50 natural derivatives have been described the structure of which has not yet been identified. It has been observed in *in vitro* studies with YTX that it can produce apoptosis, although its potency is less than that of okadaic acid, a DSP toxin that usually occurs associated to YTX (Leira, Alvarez et al., 2001); and, in contrast to what occurs with okadaic acid, YTX does not inhibit cellular phosphatases (Draisci, Lucentini et al., 2000). YTX has a cytotoxic effect because it inhibits growth in human hepatocellular carcinoma cells (HEP-G2), and it may thus be used as an antitumor drug. It has also been described that YTX modifies cytosolic calcium levels in lymphocytes (De la Rosa, L.A., Alfonso, A. et al., 2001), and increases the calcium flow induced by maitotoxin in these cells (De la Rosa, L.A., Alfonso, A. et al., 2001). It has recently been observed that YTX decreases intracellular levels of cyclic adenosine monophosphate (cAMP) second messenger through the activation of cellular phosphodiesterases (PDEs), which are the enzymes which destroy cAMP, suggesting that these enzymes may be one of the cellular targets of YTXs (Alfonso, de la Rosa et al., 2003). On the other hand, it has been observed that YTX is a histamine release inhibitor, activated by immunological stimulus, in rat mastocytes, and it may thus be used as an antiallergic or antiasthmathic drug.

In mammalian cells there are about 11 families of PDE with different isoforms (Houslay and Adams, 2003). These enzymes regulate and maintain cAMP and cyclic guanosine monophosphate (cGMP) levels constant, the latter being second messengers necessary for cell functioning involved in numerous vital functions (Soderling and Beavo, 2000). From the pharmaceutical point of view, these enzyme families are very important since their modulation is involved in treating diseases such as asthma, rheumatoid arthritis and cancer (Houslay and Adams, 2003). For this reason, describing the natural or synthetic molecules which affect PDEs and methods for studying the activity of these molecules, which may be applied in HTS (high throughput screening) protocols, are very important tools for discovering new treatments against these diseases. In this sense, describing the inhibitory effect of YTXs on tumor cell growth and the modulation they produce on histamine release are two signs of the pharmacological importance of these molecules for their possible therapeutic application.

Making use of the recent description of the YTX mechanism of action, the present invention develops methods for detecting these toxins in extracts from fishery products, based on their specific affinity for cellular PDEs. These are functional assays in which the presence of other toxins the mechanism of action of which is different does not interfere, i.e., they do not act on PDEs, but they coexist with YTX in toxic episodes. Furthermore, false positives and animal sacrifices are prevented and the contamination monitoring process in said products is expedited, since results on the exact concentration of the toxin can be obtained in 1-2 hours.

The invention set forth describes two uses based on the discovery that YTX is a PDE activator and involves the conversion of this activation into a measurable signal.

### USE 1: Method for determining PDE-YTX biomolecular bonds using an affinity sensor.

Determining molecular interactions in real time using a biosensor is a new technique the application of which is extending into different research fields (Hide, Tsutsui et al., 2002; Lee, Mozsolits et al., 2001; Mariotti, Minunni et al., 2002; Tsoi and Yang, 2002). A biosensor is used which is an equipment detecting molecular reactions between a biologically active molecule, called a ligand, and another molecule it binds to, called a receptor. The ligand is bound to the support surface of the equipment, generally a cuvette or a plate. Created on the support surface where the bonds occur is an electromagnetic field, called an evanescent field, which is extremely sensitive to changes in mass. The biosensor transforms the changes in mass occurring on the support surface due to the ligand-receptor bond into an electrical signal. Commercial biosensor models which can be used for this method are those marketed by the Biacore or Thermo Labsystems companies. In the present invention, the PDEs function as the ligand and samples with YTX, which acts as a receptor, are added thereto. The signal in the biosensor will be larger or smaller depending on the amount of toxin adhered to the PDEs and, therefore, depending on the YTX present in the sample.

Cellular PDEs which function as ligands bind to the support surface. Known concentrations of YTX, which acts as a receptor, are subsequently added on this surface. The technique works well using planar surfaces or surfaces formed by a matrix. The PDE-YTX binding follows kinetics which adjust to an equation of pseudo-first order from which a constant is obtained which is called the apparent binding rate (Rap), and which is different for each concentration of toxin. A calibration line is drawn with the Rap and toxin concentration data. A test sample (an extract of fishery products) is added on the surface, its Rap is calculated and the YTX concentration in the test sample can be obtained by placing this value on the calibration line.

### EMBODIMENT OF THE INVENTION

The method is carried out at a temperature between 22 and 37°C.
a.-A solution of PDEs at a concentration between 0.1-0.24 mg/mL at pH 7.7 is added onto an activated double compartment surface. These enzymes bind to the support surface by means of non-dissociable covalent bonds. The active groups the PDEs did not bind to were then blocked with different blocking solutions (BSA, Etanolamine, Tris-HCl...).
b.-A solution with YTX at a known concentration is added to one compartment. The other compartment is used as a blank and the toxin solvent is added into it. The association kinetics between the PDEs and YTX are recorded for 15 minutes.
c.-The ligand-receptor dissociation is then carried out by washing both compartments with buffer solution at pH 7.7, thus dissociating YTX from the PDEs.
d.-The compartments are regenerated with an acid or base solution in order to completely remove YTX. Thus the PDEs will be accessible for a new addition of YTX.
e.-Steps b, c and d are repeated with 5 different concentrations of YTX.
f.-The apparent binding rates (Rap) are obtained from the association kinetics for each concentration of toxin. The plotting of Rap values against YTX concentrations follows a linear fit with a regression coefficient greater than 0.9. A line is thus obtained with which the concentration of YTX in a sample can be obtained if its Rap is known.
g.-An extraction of the meat of the fishery product to be studied is performed. This extraction is performed following Decision 2002/225/EEC of 15 March, 2002 or any other official method (D.O.G.A., 1986) for determining maximum levels and analysis methods for certain marine toxins present in different fishery products. An aliquot of the extract (test sample) is taken and is added on the PDE bound to the surface. Association kinetics are obtained from which its Rap is calculated. By placing this test Rap value on the regression line obtained, the YTX concentration present in the sample can be determined.

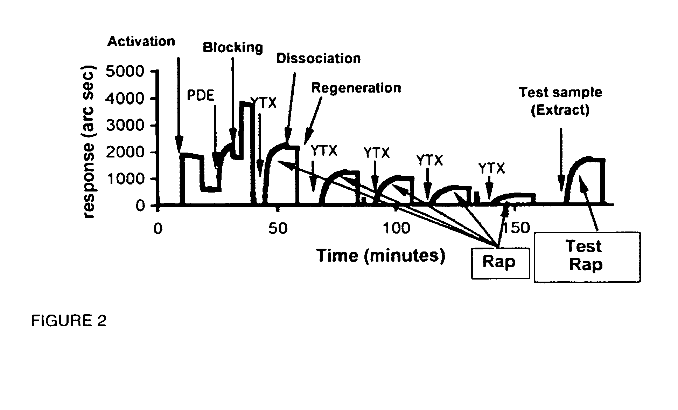

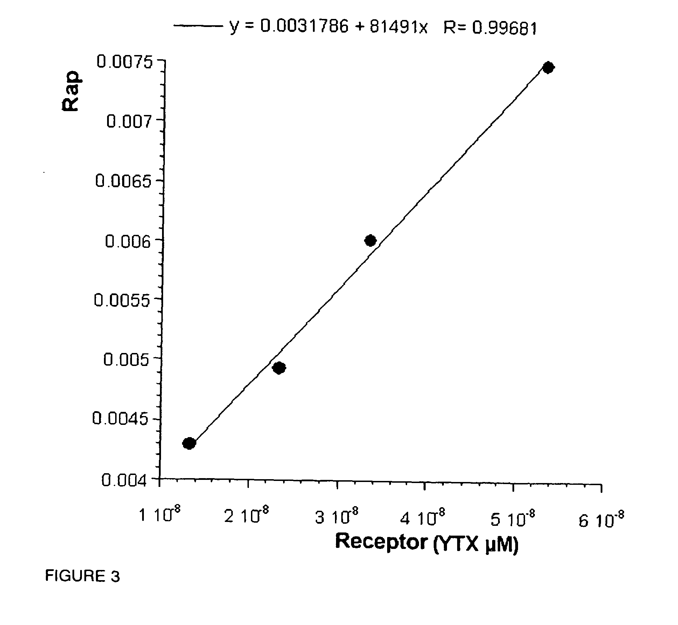

Figure 2 shows the graphic profile of the steps to be taken in this method, from surface activation to adding the test sample. The regression line is shown in Figure 3 with the Rap values against known concentrations of YTX.

### USE 2: Method for determining PDE activation by YTX using a fluorescent molecule.

A usual way of detecting cellular PDE activity is to observe their ability to destroy cAMP. There is a fluorescent derivative of cAMP, anthraniloyl-cAMP (excitation wavelength: 350 nm, emission wavelength: 445 nm), the fluorescence of which decreases as it degrades. The decrease of fluorescence over time can be expressed as the destruction rate of cAMP. In the presence of PDEs, the destruction rate increases, and if these enzymes are activated, the degradation rate will be even greater. In the present invention the degradation rate of the fluorescent indicator anthraniloyl-cAMP in the presence of PDEs is determined and its variation when samples with YTX are added is studied. Fluorescence is read with a fluorimeter that is prepared for reading microtitration plates. The destruction rate is determined in the presence of several known concentrations of YTX. The representation of the destruction rate against toxin concentration follows a linear fit with a regression coefficient greater than 0.9. A regression line is thus obtained in which the destruction rate value obtained with a sample from fishery products (test sample) can be transformed into YTX concentration.

### EMBODIMENT OF THE INVENTION

The method is carried out in a microtitration plate in a temperature range between 22 and 37°C and the fluorescence is measured at an excitation wavelength of 360 nanometers and an emission wavelength of 460 nanometers.

There are four types of wells and each one of them is carried out in duplicate.

WELLS A: Wells for calculating the cAMP concentration. Anthraniloyl-cAMP (fluorescent indicator) is added thereto at 5 concentrations between 2 and 10 µM.

WELLS B: Control wells with 8 µM of fluorescent indicator and enzymes.

WELLS C: Calibration wells with 8 µM of fluorescent indicator, enzymes and known concentrations of YTX.

WELLS D: Test sample wells with 8 µM of fluorescent indicator, enzymes and samples from an extract of any fishery product.
a.-Test buffer (10 mM Tris HCl + 1 mM CaCl₂ pH 7.4) is added in all the wells for a final incubation volume of 100µL, and the corresponding amount, depending on the type of well, of anthraniloyl-cAMP. A first reading is performed for 2 minutes.
b.-Between 2 and 5 µg of PDEs are added in wells B, C and D and a new reading is performed for 2 minutes.
c.-YTX at a known concentration or a sample from a fishery product is added to wells C and D. YTX at concentrations between 0.1 and 10 µM is added. The samples from an extract are obtained following Decision 2002/225/EEC of 15 March, 2002 or any other official method (D.O.G.A., 1986) for determining maximum levels and analysis methods for certain marine toxins present in different fishery products.
d.-After these additions the plate is shaken and successive fluorescence measurements are performed for 15 minutes, acquiring data every minute.
e.- A line is obtained with a regression coefficient greater than 0.999, by plotting the fluorescence data obtained with wells A against the concentration of indicator for each well.
f.-The fluorescence data for the rest of the wells in an anthraniloyl-cAMP concentration is transformed using the previous line in an anthraniloyl-cAMP concentration. The amount of indicator destroyed per unit of time, i.e. the destruction rate of AMPc, is obtained from the cAMP concentration at toxin addition time zero and from the concentration after 10 minutes.
g.-The destruction rate data obtained with wells B is considered as a control destruction rate.
h.-A YTX concentration standard line is obtained by plotting the destruction rate data of wells C against the YTX concentration. The YTX in that sample is determined by substituting on this line the destruction rate obtained in wells D.

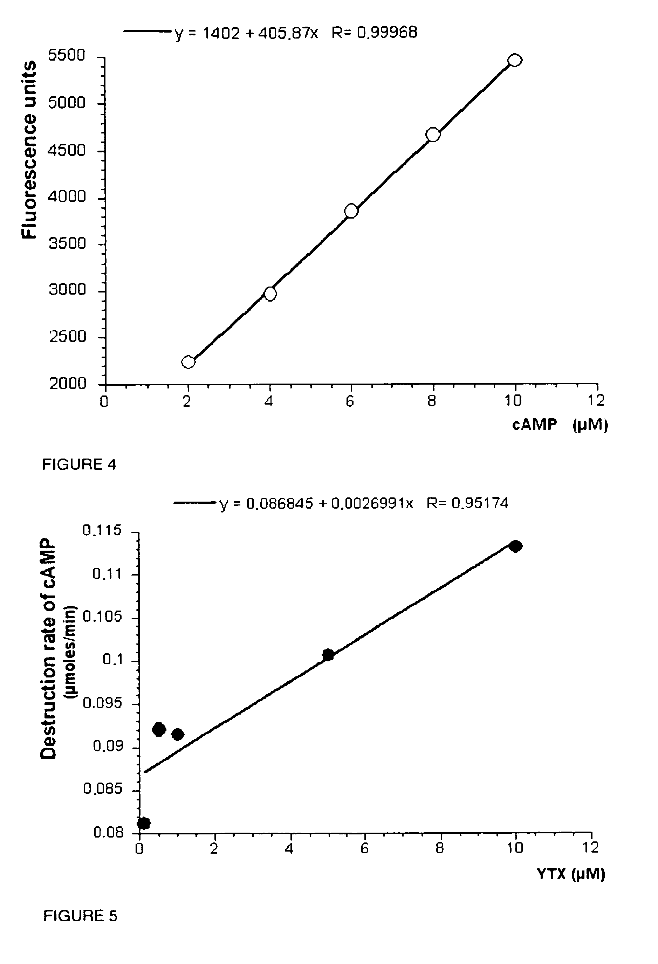

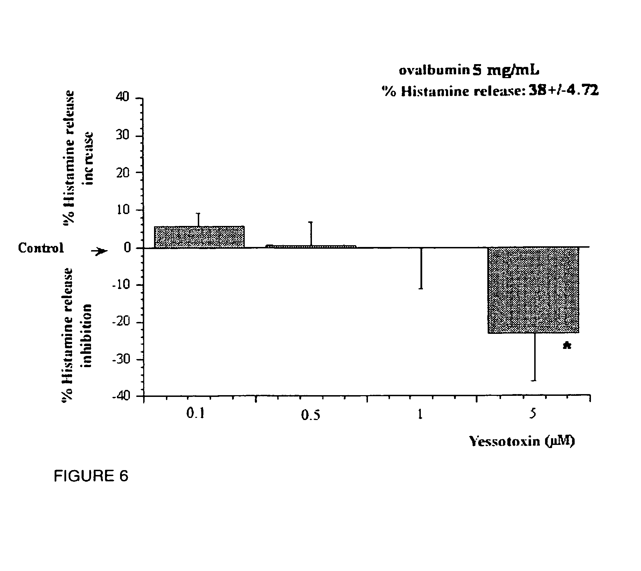

Figure 4 represents the fluorescence units calibration line against a concentration of anthraniloyl-cAMP. Figure 5 represents the standard line for destruction rates of cAMP against YTX concentration for a standard assay.

### LITERATURE

Alfonso, A., de la Rosa, L. A., Vieytes, M. R., Yasumoto, T. and Botana, L. M. (2003). "Yessotoxin a novel phycotoxin, activates phosphodiesterase activity. Effect of yessotoxin on cAMP levels in human lymphocytes." Biochem Pharmacol 65: 193-208.
D.O.G.A. (1986). "Diario oficial de Galicia".
De la Rosa, L. A., Alfonso, A., Vilariño, N., Vieytes, M. R. and Botana, L. M. (2001). "Modulation of cytosolic calcium levels of human lymphocytes by yessotoxin, a novel marine phycotoxin." Biochem. Pharmacol. 61(7): 827-833.
De la Rosa, L. A., Alfonso, A., Vilariño, N., Vieytes, M. R., Yasumoto, T. and Botana, L. M. (2001). "Maitotoxin-induced calcium entry in human lymphocytes - Modulation by yessotoxin, Ca2+ channel blockers and kinases." Cell Signal 13(10): 711-716.
Draisci, R., Lucentini, L. and Mascioni, A. (2000). Enteric toxic episodes. Pectenotoxins and yessotoxins: chemistry, toxicology, pharmacology and analysis. Seafood and freshwater toxins: pharmacology, physiology and detection. Botana, L. M. New York, Marcel Dekker: 289-324.
Draisci, R., Palleschi, L., Giannetti, L., Lucentini, L., James, K. J., Bishop, A. G., Satake, M. and Yasumoto, T. (1999). "New approach to the direct detection of known and new diarrhoeic shellfish toxins in mussels and phytoplankton by liquid chromatography-mass spectrometry." J Chromatogr A 847(1-2): 213-221.
Fernández, M. L., Míguez, A., Cacho, E., Martínez, A., Diogéne, J. and Yasumoto, T. (2002). Bioensayos con mamíferos y ensayos bioquímicos y celulares para la detección de phycotoxins. Floraciones algales nocivas en el cono sur americano. Sar, E. A., Ferrario, M. E. and Reguera, M. Madrid, UNESCO y Ministerio de Ciencia y Tecnología.
Goto, H., Igarashi, T., Yamamoto, M., Yasuda, M., Sekiguchi, R., Watai, M., Tanno, K. and Yasumoto, T. (2001). "Quantitative determination of marine toxins associated with diarrhetic shellfish poisoning by liquid chromatography coupled with mass spectrometry." J Chromatogr A 907(1-2): 181-189.
Hide, M., Tsutsui, T., Sato, H., Nishimura, T., Morimoto, K., Yamamoto, S. and Yoshizato, K. (2002). "Real-time analysis of ligand-induced cell surface and intracellular reactions of living mast cells using a surface plasmon resonance-based biosensor." Anal Biochem 302(1): 28-37.
Houslay, M. D. and Adams, D. R. (2003). "PDE4 cAMP phosphodiesterases: modular enzymes that orchestrate signalling cross-talk, desentization and compartimentalization." Biochem J. 370: 1-18.
Lee, T. H., Mozsolits, H. and Aguilar, M. I. (2001). "Measurement of the affinity of melittin for zwitterionic and anionic membranes using immobilized lipid biosensors." J Pept Res 58(6): 464-476.
Leira, F., Alvarez, C., Vieites, J. M., Vieytes, M. R. and Botana, L. M. (2001). "Okadaic acid and yessotoxin induce caspase-3 mediated apoptosis in neuroblastoma cells. Characterization of distinct apoptotic changes induced by these phycotoxins in the BE(2)-M17 cell line by means of new fluorimetric microplate assays." Toxicology in vitro 15: 277-283.
Malaguti, C., Ciminello, P., Fattorusso, E. and Rossini, G. P. (2002). "Caspase activation and death induced by yessotoxin in HeLa cells." Toxicol in Vitro 16(4): 357-363.
Mariotti, E., Minunni, M. and Mascini, M. (2002). "Surface plasmon resonance biosensor for genetically modified organisms detection." Anal Chim Acta 453(2): 165-172.
Pierotti, S., Malaguti, C., Milandri, A., Poletti, R. and Rossini, P. (2003). "Functional assay to measure yessotoxins in contaminated mussel samples." Anal Biochem 312(2): 208-216.
Ramstad, H., Larsen, S. and Aune, T. (2001). "Repeatability and validity of a fluorimetric HPLC method in the quantification of yessotoxin in blue mussels (Mytilus edulis) related to the mouse bioassay." Toxicon 39(9): 1393-1397.
Rossini, G. P. (2002). Process for measurement of dinophysistoxin and of yessotoxin. WO 02/03060 A2. International Application published under the patent cooperation treaty.
Soderling, S. H. and Beavo, J. A. (2000). "Regulation of cAMP and cGMP signaling: new phosphodiesterases and new functions." Curr. Opin. Cell. Biol. 12(174-179).
Terao, K., Ito, E., Oarada, M., Murata, M. and Yasumoto, T. (1990). "Histopathological studies on experimental marine toxin poisoning--5. The effects in mice of yessotoxin isolated from Patinopecten yessoensis and of a desulfated derivative." Toxicon 28: 1095-1104.
Tsoi, P. Y. and Yang, M. S. (2002). "Kinetic study of various binding modes between human DNA polymerase beta and different DNA substrates by surface-plasmon-resonance biosensor." Biochem J 361(2): 317-325.
Van Dolah, F. M. (2000). Diversity of marine and freshwater algal toxins. Seafood and freshwater toxins: pharmacology, physiology and detection. Botana, L. M. New York, Marcel Dekker: 19-44.
Yasumoto, T., Murata, M., Oshima, Y., Matsumoto, G. K. and Glardy, J. (1984). Diarrhetic shellfish poisoning. ACS Symp. Series. No 262. Seafood toxins. Ragelis, E. P. Washington, D.C., American Chemical Society: 207-214.
Yasumoto, T. and Takizawa, A. (1997). "Fluorometric measurement of yessotoxins in shellfish by high-pressure liquid chromatography." Biosci Biotechnol Biochem 61(10): 1775-1777.

## Claims

1. A quantitative method for detecting yessotoxis (YTXs) in fishery products based on the activation the toxins produce in cellular phosphodiesterases (PDEs) **characterized by** the use of affinity biosensors using PDEs as ligands which bond to a planar or matrix support surface which is capable of generating quantifiable molecular interactions by means of the evanescence effect in a temperature range of 20-37°C, on which YTXs are added with function as ligate, adjusting this binding to a pseudo-first order kinetics from which an apparent YTX-PDE binding rate (Rap) is obtained.

2. Quantitative method for detecting YTXs in fishery products based on the activation the toxins produce in cellular phosphodiesterases according to claim 1, **characterized by** the determination of apparent binding rates for known YTXs concentrations and the elaboration of a calibration curve where the YTX concentration of a sample from a fishery product extract which apparent binding rate is known can be calculated.

3. Quantitatives method for detecting YTXs in fishery products based on the activation the toxin produce in cellular phosphodiesterases **characterized by** detection by means of PDE activity fluorescence using anthraniloyl-cAMP as substrate, a concentration of PDEs between 2-5 µg, a temperature range of 20-37°C, a time scale of 1-15 minutes and calculating the YTX concentration of a fishery product sample from a calibration line prepared with destruction rates of the fluorescent substrate obtained for known concentrations of YTX.

4. A quantitative method for detecting YTXs in fishery products based on the activation the toxin produce in cellular phosphodiesterases according to claim 3, **characterized by** determining the change in intensity of polarized fluorescence taking place when YTX binds to PDEs.

## Patentansprüche

1. Quantitatives Verfahren zum Nachweisen von Yessotoxinen (YTXs) in Fischereiprodukten, welches auf der Aktivierung beruht, die die Toxine bei zellulären Phosphodiesterasen (PDEs) hervorrufen, **gekennzeichnet durch** die Verwendung von Affinitätsbiosensoren, die PDEs als Liganden verwenden, welche an eine planare oder Matrixträgeroberfläche binden, welche in der Lage ist, quantifizierbare molekulare Wechselwirkungen mittels des Evaneszenzeffekts in einem Temperaturbereich von 20 bis 37 °C zu generieren, auf welche YTXs mit der Funktion als Ligat zugegeben werden, Anpassen dieser Bindung an eine Kinetik der pseudoersten Ordnung, aus der eine scheinbare YTX-PDE-Bindungsrate (Rap) erhalten wird.

2. Quantitatives Verfahren zum Nachweisen von YTXs in Fischereiprodukten, welches auf der Aktivierung beruht, die die Toxine in zellulären Phosphodiesterasen hervorrufen, nach Anspruch 1, **gekennzeichnet durch** die Bestimmung der scheinbaren Bindungsraten für bekannte YTXs-Konzentrationen und die Ausarbeitung einer Eichkurve, wo die YTX-Konzentration einer Probe aus einem Extrakt eines Fischereiprodukts, dessen scheinbare Bindungsrate bekannt ist, berechnet werden kann.

3. Quantitatives Verfahren zum Nachweisen von YTXs in Fischereiprodukten, welches auf der Aktivierung beruht, die das Toxin bei zellulären Phosphodiesterasen hervorruft, **gekennzeichnet durch** die Bestimmung der Fluoreszenz der PDE-Aktivität unter Verwendung von Anthraniloyl-cAMP als Substrat, eine Konzentration von PDEs zwischen 2 bis 5 µg, einen Temperaturbereich von 20 bis 37 °C, einen Zeitmaßstab von 1 bis 15 Minuten und Berechnen der YTX-Konzentration einer Probe eines Fischereiprodukts aus einer Eichlinie, die mit Zerfallsraten des fluoreszierenden Substrats hergestellt wurde, welche für bekannte Konzentrationen von YTX erhalten wurden.

4. Quantitatives Verfahren zum Nachweisen von YTXs in Fischereiprodukten, welches auf der Aktivierung beruht, die das Toxin bei zellulären Phosphodiesterasen hervorruft, gemäß Anspruch 3, **gekennzeichnet durch** das Bestimmen der Veränderung der Intensität der polarisierten Fluoreszenz, welche auftritt, wenn YTX an PDEs bindet.

## Revendications

1. Méthode quantitative de détection de yessotoxines (YTX) dans des produits halieutiques (PDE) basée sur l'activation causée par les toxines dans les phosphodiestérases cellulaires (PDE), **caractérisée par** l'utilisation de biocapteurs d'affinité utilisant des PDE comme ligands qui se lient à une surface de support plane ou de.matrice qui est capable de générer des interactions moléculaires quantifiables au moyen de l'effet d'évanescence dans une plage de températures de 20 à 37°C, sur laquelle les YTX sont ajoutées qui fonctionnent comme ligand, ajustant cette liaison à une cinétique de pseudo premier ordre à partir de laquelle un taux de liaison YTX-PDE apparent (Rap) est obtenu.

2. Méthode quantitative de détection de YTX dans des produits halieutiques basée sur l'activation causée par les toxines dans les phosphodiestérases cellulaires selon la revendication 1, **caractérisée par** la détermination de taux de liaison apparents pour les concentrations connues de YTX et l'élaboration d'une courbe de calibrage dans laquelle la concentration en YTX d'un échantillon issu d'un extrait de produit halieutique dont le taux de liaison apparent est connu peut être calculée.

3. Méthode quantitative de détection de YTX dans des produits halieutiques basée sur l'activation causée par les toxines dans les photodiestérases cellulaires **caractérisée par** la détection au moyen de l'activité fluorescence des PDE en utilisant l'anthraniloyl-cAMP comme substrat, une concentration des PDE entre 2-5 µg, une plage de températures de 20-37°C, une échelle de temps de 1-15 minutes et en calculant la concentration en YTX d'un échantillon de produit halieutique à partir d'une ligne de calibrage préparée avec les taux de destruction du substrat fluorescent obtenus pour des concentrations connues de YTX.

4. Méthode quantitative de détection des YTX dans des produits halieutiques basée sur l'activation causée par les toxines dans les phosphodiestérases cellulaires selon la revendication 3, **caractérisée par** la détermination du changement d'intensité de la fluorescence polarisée ayant lieu lorsque la YTX se lie aux PDE.
